# EUROPEAN PATENT SPECIFICATION

(11) **EP 3 086 664 B1**
(45) Date of publication and mention of the grant of the patent: **15.07.2020**
(21) Application number: 13821384.8
(22) Date of filing: 26.12.2013
(51) Int. Cl.: A23K 10/18, A23K 20/174, A23K 20/20, A23K 50/40, A23L 33/14, C12N 1/16

(54) **METHOD OF PREPARING NUTRITIONALLY FORTIFIED YEAST**
VERFAHREN ZUR HERSTELLUNG VON MIT NÄHRSTOFFEN ANGEREICHERTER HEFE
MÉTHODE DE PRÉPARATION DE LEVURE ENRICHIE NUTRITIONNELLEMENT

(43) Date of publication of application: 02.11.2016
(73) Proprietor: Hill's Pet Nutrition, Inc., Topeka, KS 66603 (US); AB Mauri Food Inc. D/B/A Ohly Americas, Hutchinson, Minnesota 55350 (US)
(72) Inventor: JEWELL, Dennis, Lawrence, Kansas 66049 (US); LIN, Hungwei, Lawrence, Kansas 66049 (US); GOSHAW, David, Hutchinson, Minnesota 55350 (US)
(74) Representative: Chapman, Paul Gilmour
(86) International application number: PCT/US2013/077775
(87) International publication number: WO 2015/099727

(56) References cited:
- BE-A3- 1 019 927
- CN-A- 101 361 567
- US-A- 4 118 512
- US-A- 4 265 913
- US-A1- 2011 003 035
- GUNNARD K JACOBSON: "Focus on Dietary supplements & contract services 14 Supplement to AgroFood industry hi- Selenium anD human SelenoproteinS", AGROFOOD INDUSTRY HI-TECH, vol. 21, 1 July 2010 (2010-07-01), pages 14-16, XP055125877,
- Anonymous: "Levures nutritionnelles et enrichies, protéines, vitamines B et régimes végétariens", , 1 January 2009 (2009-01-01), XP055125949, Retrieved from the Internet: URL:http://www.lesaffrehumancare.fr/ingred ients-nutrition-sante/ingredients-nutritio n-sante/levures-nutritionnelles-et-enrichi es.html [retrieved on 2014-06-30]

## Description

### FIELD OF INVENTION

The present invention relates to a method of preparing nutritionally fortified yeast, a nutritionally fortified yeast composition and a food composition.

The invention is defined by the appended claims.

### BACKGROUND

Yeast is often used in the food manufacturing industry, and particularly in the pet food manufacturing industry as a source of protein, nutraceutical supplement, texture modifier, and aroma enhancer. Furthermore, yeast is often used as a palatability enhancer due to the high inherent content of free glutamate and nucleotides.

There is a broad spectrum of yeast species that is used in the food industry. The most common yeast species currently utilized by the pet food industry include *Saccharomyces cerevisiae* (also known as baker's yeast or brewer's yeast) and *Torulopsis utilis, Torula utilis,* or *Candida utilis* (also known as Torula yeast).

In addition to the abundant source of protein, glutamate and nucleotides, yeast also contains micronutrients such as B vitamins, potassium, and selenium, which can be used to supplement the diets of pets. It is preferable to utilize yeast as a source of micronutrients for pet foods rather than incorporating micronutrients individually into pet foods, to simplify the manufacturing process and to simplify long ingredient lists on pet food labels, which may deter pet owners.

It would therefore be desirable to provide a method of preparing a nutritionally fortified yeast with a high level of fortification which could be used as an effective vehicle for supplementing foods with micronutrients.

### BRIEF SUMMARY

The present inventors have found that when micronutrients are combined with yeast post-fermentation, an unexpectedly high level of micronutrient retention occurs.

Accordingly, in a first aspect, the present invention provides a method of preparing nutritionally fortified yeast comprising:
a) incubating yeast with at least one fermentable substrate under conditions suitable for achieving fermentation;
b) isolating the yeast after fermentation;
c) combining the isolated yeast with one or more chelated minerals and at least one micronutrient selected from vitamins, amino acids and antioxidants to obtain nutritionally fortified yeast; and
d) drying the nutritionally fortified yeast.

The isolated yeast may be combined with at least one vitamin and chelated mineral. Alternatively, the isolated yeast may be combined with at least one chelated mineral, in the absence of any vitamins.

Isolated yeast is combined with one or more minerals that are chelated. Preferably, the chelant comprises a peptide or partially hydrolysed peptide. More preferably, the one or more minerals are selected from a proteinate of calcium, potassium, sodium, magnesium, iron, copper, manganese, zinc, iodine, cobalt and selenium.

Typically in the method of the present invention, the vitamins comprise vitamin A, vitamin C, vitamin D, vitamin E, vitamin B1(thiamin), vitamin B2 (riboflavin), vitamin B3 (niacin), vitamin B5 (pantothenic acid), vitamin B6 (pyridoxine), vitamin B9 (folic acid), vitamin B12 (cobalamin), and choline.

Optionally, the isolated yeast is combined with the one or more chelated mineral and at least one micronutrient selected from vitamins, amino acids and antioxidants at a temperature of 3°C to 45°C for a period of 30 minutes to 48 hours.

Typically in the method of the present invention, the minerals comprise calcium, potassium, sodium, magnesium, iron, copper, manganese, zinc, iodine, cobalt and selenium. The minerals are chelated as defined above.

Typically in the method of the present invention, the amino acids comprise threonine, isoleucine, lysine, methionine, cystine, phenylalanine, tyrosine, valine, arginine, histidine, alanine, and aspartic acid.

Typically in the method of the present invention, the antioxidants comprise taurine, lipoic acid, glutathione, N-acetyl cysteine, vitamin E, vitamin C and beta-carotene.

Optionally, the isolated yeast is combined with at least one vitamin and chelated mineral. Further optionally, the isolated yeast is combined with a chelated mineral and a heat-labile vitamin such as vitamin B1 (thiamin) and/or vitamin C. Still further optionally, the isolated yeast is combined with at least one chelated mineral selected from iron, zinc, copper, and manganese.

Typically, in step c) the isolated yeast comprises a yeast cream. Optionally, the yeast cream comprises 15 wt.% to 25 wt.% dry matter.

Preferably, the isolated yeast is combined with at least one vitamin in a total amount of 0.1 wt.% to 2 wt.% vitamins by total weight of the solid content of the isolated yeast. More preferably, the isolated yeast is combined with vitamins in a total amount of 1 wt.% to 1.5 wt.% vitamins by total weight of the solid content of the isolated yeast.

Preferably, the isolated yeast is combined with minerals in a total amount of 0.01 wt.% to 2 wt.% minerals by total weight of the solid content of the isolated yeast. More preferably, the isolated yeast is combined with minerals in a total amount of 0.1 wt.% to 1 wt.% minerals by total weight of the solid content of the isolated yeast. The minerals are chelated as defined above.

Typically, in step b), the yeast is isolated by centrifugation. Preferably, the isolated yeast is allowed to autolyse prior to combining with the at least one micronutrient. More preferably, the autolysed yeast comprises 10 to 25 wt.% dry matter.

Typically in the method of the present invention, the nutritionally fortified yeast is pasteurized by heating to a temperature of 60°C to 135°C. Optionally, the nutritionally fortified yeast is dried to a moisture content of 1 wt.% to 10 wt.%. Further optionally, the yeast is dried by spray-drying or freeze-drying.

Typically, the fermentable substrate comprises ethanol. Optionally, the yeast is *Torulopsis utilis, Torula utilis* or *Candida utilis.*

A nutritionally fortified yeast composition is obtained by the method as defined herein.

A food composition can be obtained comprising nutritionally fortified yeast obtained by the method as defined herein at least one food ingredient. Optionally, the food composition comprises the nutritionally fortified yeast in an amount of up to 2 wt.%.

The present invention provides a use of a nutritionally fortified yeast composition obtained by the method as defined herein as a palatability enhancer.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### DETAILED DESCRIPTION

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

As used herein, the term "food" may refer not only to a food product which typically provides most, if not all, the nutrient value for an animal, but may also refer to such items as a snack, treat, and supplement.

In some embodiments, the present invention provides a method of preparing nutritionally fortified yeast comprising:
a) incubating yeast with at least one fermentable substrate under conditions suitable for achieving fermentation;
b) isolating the yeast after fermentation;
c) combining the isolated yeast with one or more chelated minerals and at least one micronutrient selected from vitamins, amino acids and antioxidants to obtain nutritionally fortified yeast; and
d) drying the nutritionally fortified yeast.

### Yeast

A variety of species of yeast may be used in the methods of the present invention. These include without limitation, *Saccharomcyes, Kluyveromyces, Candida* and *Torulaspora.* In one embodiment, the yeast is *Saccharomcyes cerevisiae.* In a preferred embodiment, the yeast is *Torulopsis utilis, Torula utilis* or *Candida utilis* (Torula yeast). Torula yeast is a good candidate for the methods of the present invention due to its inherently low selenium level (which can cause toxicity in the consumer).

In the methods of the present invention, yeast is fermented according to standard methods that would be known to those skilled in the art. Fermentable substrates include dextrose, glucose, maltose, fructose and sucrose. In one embodiment the fermentable substrate is ethanol. Ethanol is particularly suitable for Torula yeast fermentation.

Typically, after fermentation, the yeast is isolated. By "isolation" it is meant that the yeast is separated from a major proportion of the fermentation broth. The yeast is preferably isolated by centrifugation. The centrifugate may be washed and re-centrifuged to yield isolated yeast. In some embodiments, the isolated yeast is a yeast cream having a solid content of about 15 wt.% to about 25 wt.%. Preferably, the solid content of the yeast cream is from about 15 wt.%, 16 wt.%, 17 wt.% or 18 wt.% to about 22 wt.%.

In one embodiment, the yeast isolated from the fermentation broth, for example, in the form of a yeast cream, is combined with one or more micronutrients for fortification of the yeast as described below. In another embodiment, yeast extract may be prepared from the isolated yeast. Conventional methods of preparing yeast extract would be known to the person skilled in the art of food manufacturing. Typically methods involve removing cells walls and isolating the cellular contents. In yet another embodiment, the isolated yeast is allowed to autolyse. Yeast autolysis is a complex and slow phenomenon which involves the yeast's endogenous hydrolytic enzymes such as proteases, nucleases, lipases and glycanases degrading cellular components. Unlike the preparation of yeast extract, cell wall components are not removed during autolysis. Optimal conditions for performing autolysis would be known to the person skilled in the art of food manufacturing. In one embodiment, the solid content (dry matter) of the autolysed yeast is from 10 wt.% or 15 wt.% to 25 wt.%. Often the solid content (dry matter) of the autolysed yeast is from about 15 wt.%, 16 wt.%, 17 wt.% or 18 wt.% to about 22 wt.%. The autolysed yeast extract may subsequently be combined with one or more micronutrients for fortification of the yeast as described below. In one embodiment, a mixture of (unautolysed) yeast cream and autolysed yeast is fortified with micronutrients.

### Micronutrients

The isolated yeast is combined with one or more chelated minerals and at least one micronutrients selected from vitamins, minerals, amino acids and antioxidants. Preferably, the isolated yeast is combined with at least one vitamin and at least one chelated mineral.

Vitamins include without limitation, vitamin A, vitamin B1 (thiamine), vitamin B2 (riboflavin), vitamin B3 (niacin), vitamin B5 (pantothenic acid), vitamin B6 (pyridoxine), vitamin B9 (folic acid), vitamin B12, (cobalamin), biotin, choline vitamin C, vitamin D and vitamin E.

In some embodiments, the isolated yeast is combined with a heat-labile vitamin such as vitamin B1 (thiamine) and/or vitamin C. The present inventors have found that a high level of fortification is observed even with heat-labile vitamins.

One or more vitamins may be combined with the isolated yeast in a total amount of 0.1 wt.% to 5 wt.%, or 0.1 wt.% to 4 wt.%, or 0.1 wt.% to 3 wt.%, or 0.1 wt.% to 2 wt.% vitamins by total weight of the solid content(dry matter) of the isolated yeast. In a preferred embodiment, the total amount of vitamins is 0.5 wt.% to 3 wt.%, or 0.5 wt.% to 2 wt.%, or 0.5 wt.% to 1 wt.%, or 1 wt.% to 3 wt.%, or 1 wt.% to 2 wt.% by total weight of the solid content (dry matter) of the isolated yeast.

Minerals include without limitation, calcium, potassium, sodium, magnesium, iron, copper, manganese, zinc, iodine, cobalt and selenium. In one embodiment, the isolated yeast is combined with at least one mineral selected from iron, zinc, copper and manganese.

One or more minerals may be combined with the isolated yeast in a total amount of 0.1 wt.% to 3 wt.%, 0.1 wt.% to 2 wt.%, or 0.1 wt.% to 1 wt.%, or 0.1 wt.% to 0.5 wt.% minerals by total weight of the solid content (dry matter) of the isolated yeast. In a preferred embodiment, the total amount of minerals is 0.5 wt.% to 3 wt.%, or 0.5 wt.% to 2 wt.%, or 0.5 wt.% to 1 wt.% by total weight of the solid content (dry matter) of the isolated yeast.

The minerals are chelated. Thus, the isolated yeast is combined with one or more chelated minerals. The minerals may be chelated by one or more amino acids, a partially hydrolysed peptide, or a peptide. Chelated minerals that may be used in the methods of the present invention include without limitation, a proteinate of calcium, phosphorus, potassium, sodium, chloride, magnesium iron, copper, manganese, zinc, iodine, and selenium. Chelated minerals are commercially available from various sources such as Alltech® (Nicholasville KY). In some embodiments, at least two minerals may be chelated by the same chelant as a complex structure. One example of such a multimineral-chelant complex is Bioplex® zinc/copper/manganese.

The present inventors have found that when conventional (unchelated) minerals are used in the methods of the present invention, the minerals have a tendency to coagulate and precipitate with yeast. This significantly reduces the level of fortification. Such coagulation and precipitation is not observed with chelated minerals, and the level of fortification is also increased with chelated minerals.

Amino acids include without limitation asparagine, glutamine, histidine, isoleucine, leucine, lysine, methionine, phenylalanine, threonine, tryptophan, alanine, arginine, aspartic acid cysteine, glutamic acid, glutamine, glycine, tyrosine, proline, valine and serine. In one arrangement, the amino acid is selected from threonine, isoleucine, lysine, methionine, cystine, phenylalanine, tyrosine, valine, arginine, histidine, alanine, and aspartic acid.

One or more amino acids may be combined with the isolated yeast in a total amount of up to 1 wt.% by total weight of the solid content (dry matter) of the isolated yeast. In some embodiments, one or more amino acids are combined with the isolated yeast in a total amount of 0.1 wt.% to 1 wt.%, or 0.1 wt.% to 0.5 wt.% or 0.1 wt.% to 0.3 wt.% by total weight of the solid content (dry matter) of the isolated yeast. In other embodiments, one or more amino acids are combined with the isolated yeast in a total amount of 0.3 wt.% to 1 wt.%, 0.5 to 1 wt.% or 0.5 wt.% to 0.8 wt.% by total weight of the solid content (dry matter) of the isolated yeast.

Antioxidants include without limitation, taurine, lipoic acid, glutathione, N-acetyl cysteine, vitamin E, vitamin C and beta-carotene. Preferably, the antioxidant is taurine.

One or more antioxidants may be combined with the isolated yeast in a total amount of up to 0.2 wt.% by total weight of the solid content (dry matter) of the isolated yeast. In one embodiment, one or more antioxidants are combined with the isolated yeast in a total amount of 0.01 wt.% to 0.2 wt.%, or 0.01 wt.% to 0.1 wt.% or 0.01 wt.% to 0.05 wt.% by total weight of the solid content (dry matter) of the isolated yeast. In another embodiment, one or more antioxidants are combined with the isolated yeast in a total amount of 0.05 wt.% to 0.2 wt.%, or 0.05 wt.% to 0.1 wt.% or 0.05 wt.% to 0.08 wt.% by total weight of the solid content (dry matter) of the isolated yeast.

Typically the micronutrients are combined with the isolated yeast at a temperature of 3°C to 45°C for a period of 0.5 hours to 48 hours. In some embodiments, the micronutrients are combined with the isolated yeast at a temperature of 5°C to 25°C or 5°C to 10°C. In other embodiments, the micronutrients are combined with the isolated yeast at a temperature of 25°C to 45°C or 10°C to 20°C. Preferably, the micronutrients are combined with the isolated yeast for a period of from 0.5 hours, 1 hour, 5 hours, 10 hours or 20 hours up to 30 hours. In one embodiment, the micronutrients are combined with the isolated yeast for a period of 10 hours to 20 hours. As mentioned above, the isolated yeast typically has a solid content ranging from about 15 wt.% to about 22 wt.%. Fermentation media constitutes the remainder of the isolated yeast. In some embodiments, micronutrients are combined directly with the isolated yeast. Thus, micronutrients may be combined directly with the isolated yeast in solid form, without being made into a solution first. During step c) as described herein, the yeast and micronutrients may be mixed continuously to ensure optimal fortification.

Without being bound by theory, it is thought that the micronutrients may become adhered to or encapsulated by yeast cellular components, resulting in the fortification of the yeast.

It is advantageous to combine the micronutrients with yeast after fermentation and isolation of the yeast, as the incorporation of micronutrients into the fermentation media during fermentation may interfere with the fermentation process.

### Pasteurization

After combining the micronutrients with the isolated yeast to form nutritionally fortified yeast, the nutritionally fortified yeast may be pasteurized and/or dried. Methods of pasteurization and drying would be known to those skilled in the art of food manufacturing. Typically, the nutritionally fortified yeast is heated to a temperature of 60°C to 135°C, or from 80°C to 100°C, or from 90°C to 95°C. The time period over which the nutritionally fortified yeast is heated may be from 5 seconds to 200 seconds, or from 5 seconds to 120 seconds, or from 30 seconds to 120 minutes.

The nutritionally fortified yeast may be dried by conventional techniques such as spray-drying or freeze-drying. Typically, the nutritionally fortified yeast is dried to a moisture content of 0.5 wt.% to 10 wt.%, or from 1 wt.% to 5 wt.% or from 1 wt.% to 2 wt.% by total weight of the yeast.

### Uses of nutritionally fortified yeast

The present inventors have found that yeast fortified according to the methods of the present invention unexpectedly retain high levels of micronutrients. The term "retain" or "retention" as used herein refers to an association or combination of micronutrients with the yeast or yeast cellular components. Therefore, nutritionally fortified yeast obtained by the methods of the present invention is useful for incorporation into food compositions, and in particular, pet food compositions.

Given the high level of micronutrient retention that is achieved by the methods of the present invention, an amount as low as 4 wt.%, 3 wt.% or 2 wt.% of the nutritionally fortified yeast may be incorporated into food compositions to achieve adequate micronutrient supplementation of the food. Thus, in some embodiments, the food composition comprises a nutritionally fortified yeast obtained by the methods of the present invention in amount of up to 4 wt.%, up to 3 wt.%, or up to 2 wt.% by total weight of the composition. In other embodiments, the food composition comprises a nutritionally fortified yeast obtained by the methods of the present invention in amount of 1 wt.% to 3 wt.% or 1 wt.% to 2 wt.% by total weight of the composition.

The food composition typically comprises nutritionally fortified yeast and at least one food ingredient. The at least one food ingredient may be selected from protein (for example, meat, meat-by products, dairy products, eggs, wheat protein, soy protein and potato concentrate), fat (for example, animal fat, fish oil, vegetable oil, meat and meat by-products), and carbohydrate (for example, grains such as wheat, corn, barley and rice). Other food ingredients include, without limitation, fiber (for example cellulose, beet pulp, peanut hulls and soy fiber), vitamins, minerals and preservatives. The food ingredient may be any food ingredient defined herein.

In some embodiments, the food compositions comprise nutritionally fortified yeast obtained by the method as defined herein and at least one food ingredient selected from protein, fat and carbohydrate. Food compositions supplemented with nutritionally fortified yeast obtained by the method of the present invention may comprise 0.01 wt.% to 0.2 wt.% vitamin C, 0.01 wt.% to 0.2 wt.% vitamin E, 0.005 wt.% to 0.05 wt.% thiamine, 0.005 wt.% to 0.2 wt.% zinc and 0.002 wt.% to 0.1 wt.% iron.

Table 1 indicates a typical micronutrient profile of a food composition comprising nutritionally fortified yeast, wherein the micronutrients are derived from the nutritionally fortified yeast.

**Table 1 - Micronutrient profile of food composition comprising up to 2 wt.% nutritionally fortified yeast.**

| Mineral | Unit | Min. | Max. |
|---|---|---|---|
| CALCIUM | % | 0.050 | 1.200 |
| POTASSIUM | % | 0.050 | 1.200 |
| MANGANESE | ppm | 50 | 1,000 |
| SODIUM | % | 0.050 | 1.2 |
| MAGNESIUM | % | 0.050 | 0.600 |
| IRON | ppm | 10 | 4,000 |
| SELENIUM | ppm | 0.05 | 500 |
| COBALT | ppm | 0.1 | 1000 |
| IODINE | ppm | 0.1 | 1,000 |
| ZINC | ppm | 50 | 2000 |

| Vitamin | Unit | Min. | Max. |
|---|---|---|---|
| VITAMIN A | IU/Kg | 1,000 | 500,000 |
| VITAMIN D (TOT) | IU/Kg | 100 | 4,000 |
| VITAMIN E (TOT) | IU/Kg | 50 | 3,000 |
| VITAMIN C | ppm | 5.000 | 1000 |
| FOLIC ACID | ppm | 0.01 | 1000 |
| RIBOFLAVIN | ppm | 1 | 1000 |
| THIAMINE | ppm | 1 | 1000 |
| PYRIDOXINE | ppm | 1 | 1000 |
| VIT B12 | ppm | 0.01 | 1000 |

The food composition may be suitable for consumption by any animal. Preferably, the food composition is for consumption by non-human animals, which include, without limitation, avians, bovines, canines, equines, felines, murines, ovines, and porcines.

More preferably, the food composition is for consumption by a pet. Most preferably, the food composition is for consumption by a companion animal, including a canine or a feline.

Food compositions can be prepared in a dry or wet form using conventional processes.

Given that the methods of the present invention result in high levels of micronutrient retention in the nutritionally fortified yeast, in some embodiments, the food composition as defined herein is free of further supplemented or exogenously incorporated micronutrients (for example, vitamins and minerals). The term "supplemented or exogenously incorporated micronutrients" refers to micronutrients that are not contained with the at least one food ingredient of the food composition or the nutritionally modified yeast.

In yet another arrangement, the nutritionally fortified yeast obtained by the methods of the present invention may be used to enhance the palatability of a food composition. Accordingly, the present invention further provides a use of nutritionally fortified yeast obtained by the method defined herein as a palatability enhancer.

The invention is further illustrated in the following non-limiting Examples.

### EXAMPLES

### Example 1 - Fortification of yeast with thiamine (vitamin B1) - not according to the invention.

In order to assess micronutrient retention by yeast according to the method of the present invention, Torula yeast was fermented in the presence of ethanol. Post-fermentation, the yeast was centrifuged to 15 % to 22% solids, cooled, and placed in a process tank. A portion of the yeast was subjected to autolysis in this tank and the remainder was retained in the tank as (unautolysed) yeast cream. When the autolysis process was completed, the yeast cream and autolysed yeast were mixed to form an isolated yeast mixture. Subsequently, the isolated yeast mixture was combined and mixed with four different amounts of a vitamin blend comprising amongst other vitamins, 4.35 wt.% thiamine, as indicated in Table 2. The mixing took place in a high speed, high sheer mixer over a period of at least 30 minutes and at a temperature in the range of 20°C-30°C. Subsequently, the fortified yeast was pasteurized.

The amount of thiamine retained in the yeast after fortification was subsequently determined by using acid hydrolysis to promote the release of the thiamine from the yeast matrix, followed by HPLC determination for separation and quantitative determination of the thiamine. The determined amount of thiamine was subsequently compared to expected thiamine retention levels (assuming no losses). Thiamine was selected as the prototype vitamin as it is susceptible to thermal degradation, and thus it is likely to be more susceptible to a reduced recovery. The results are illustrated in Table 2. As detailed below, all calculations are conducted on a dry matter basis to account for moisture variability.

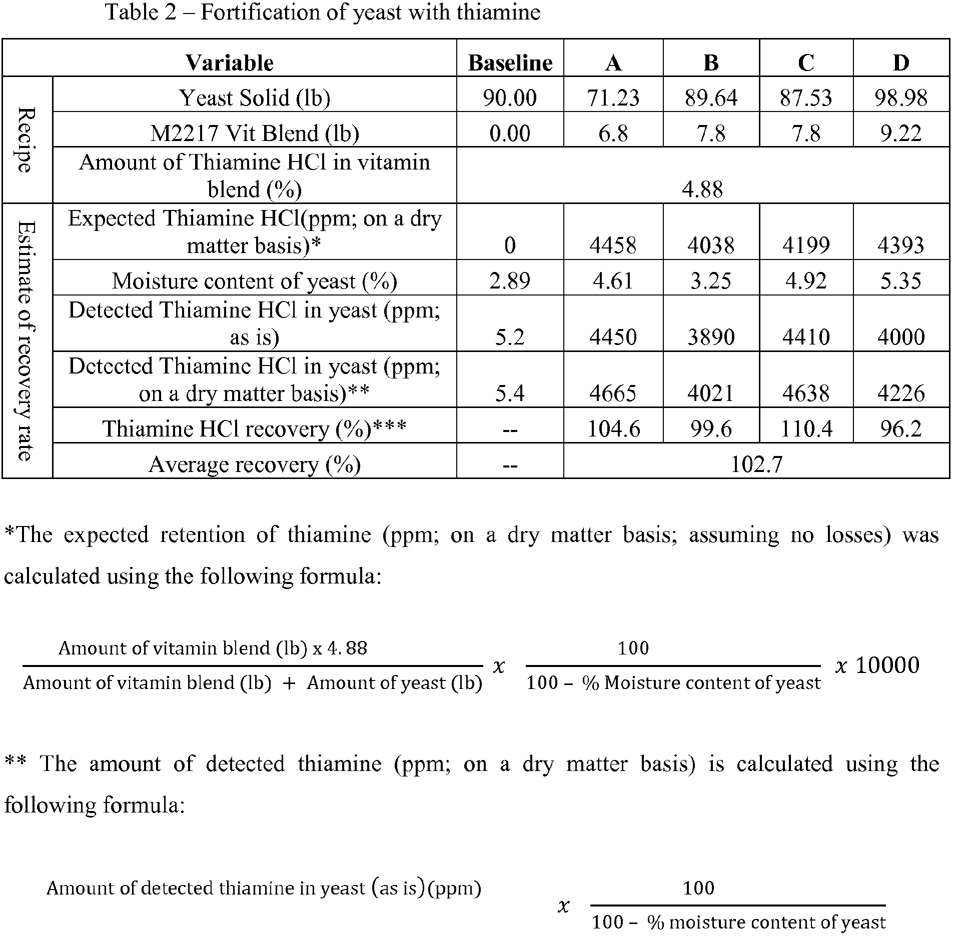

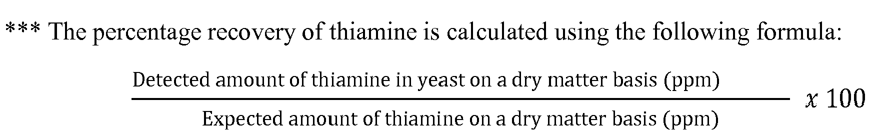

As can be seen from Table 2, at all thiamine concentrations tested, there was approximately 100% retention of micronutrients. As discussed above, "retention" relates to the association or combination of micronutrients with the yeast or yeast cellular components. Thus, a 100% retention signifies that there is no loss in micronutrients during the fortification process. (In this experiment, values greater than 100% could be attributable to experimental error). Thus, the fortification process is highly effective and would be suitable for other vitamins.

### Example 2 - Fortification of yeast with minerals - not according to the invention.

The method set out in Example 1 was repeated with various minerals as set out in Table 3. The minerals were combined with yeast either alone, or with the vitamin pre-mix. The amount of minerals retained during the fortification was determined by converting the target elements into ashes by combustion of organic substances in the yeast matrix, followed by quantitatively determining the target elements using an Inductively Coupled Plasma instrument.

**Table 3 - Fortification of yeast with minerals**

| **Variable** | **Sample No** | **Sample Size (n)** | **Zn (ppm)** | | | **Mn (ppm)** | | |
|---|---|---|---|---|---|---|---|---|
| | | | Exp* | Act** | Rec*** | Exp* | Act** | Rec*** |
| Mineral | DOE1-7 | 3 | 11,550 | 5,069 | 43.9% | 550 | 427 | 77.5% |
| Combo | DOE1-8 | 2 | 9,010 | 3,903 | 43.3% | 450 | 465 | 103.3% |
| | | | | | | | | |

| **Variable** | **Sample No** | **Sample Size(n)** | **Fe (ppm)** | | | **Cu (ppm)** | | |
|---|---|---|---|---|---|---|---|---|
| | | | Exp* | Act** | Rec*** | Exp* | Act** | Rec*** |
| Mineral | DOE1-7 | 3 | 11,550 | 1,914 | 16.6% | 577 | 456 | 79.1% |
| Combo | DOE1-8 | 2 | 9,010 | 2,863 | 31.8% | 430 | 640 | 148.8% |

| | | | | | | | | |
|---|---|---|---|---|---|---|---|---|
| * = Expected recovery (if no losses) ** = Actual amount recovered *** = % recovery (retention) | | | | | | | | |

As can be seen in Table 3, the retention values varied significantly with the mineral tested. Manganese and copper were most effectively retained during the fortification process with retention values of ∼78% and ∼79%, respectively. However, the retention values for zinc and iron were significantly lower (∼44% and ∼17%, respectively). Additionally, during the fortification process, there was a noticeable precipitation/coagulation of the minerals. This may have accounted for the low/variable retention rates.

### Example 3 -Fortification with vitamins and chelated minerals

The method set out in Example 1 was repeated using thiamine and vitamin C (as individual vitamins rather than a vitamin blend), and various chelated minerals. Chelated minerals (Bioplex® zinc/manganese/copper and Bioplex® iron) were purchased from Alltech® (Nicholasville KY). The fortification process was carried out using the minerals alone, vitamins alone, and both vitamins and minerals. The retention of vitamins and minerals was quantified as described in Examples 1 and 2, respectively.

The amounts of vitamins/chelated minerals used in the fortification process and the expected retention values (as calculated using the above equations) are set out in Table 4. The results of the experiment are provided in Tables 5 and 6.

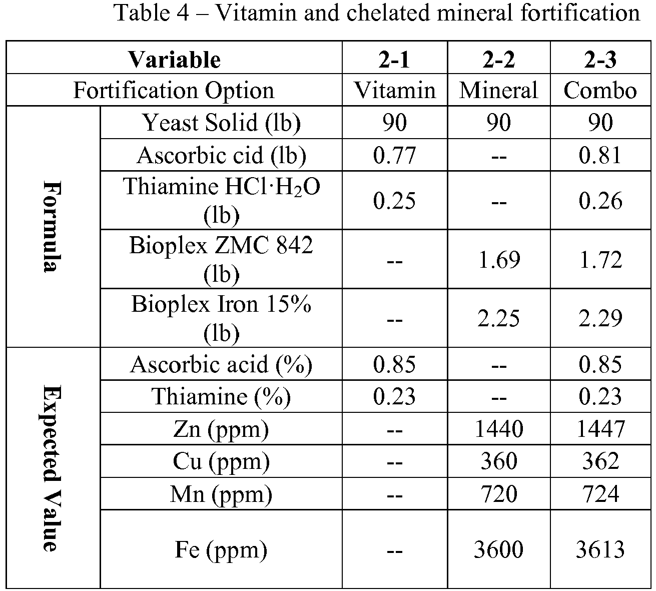

**Table 5 - Retention of vitamin B1 and vitamin C in yeast**

| Variable | Sample No | Vit C (%) | | | Vit B1 (%) | | |
|---|---|---|---|---|---|---|---|
| | | Expected | Actual | Recovery | Expected | Actual | Recovery |
| Vitamin | DOE2-1 | 0.85 | 0.354 | 41.65% | 0.23 | 0.319 | 138.70% |
| Combo | DOE2-3 | 0.85 | 0.34 | 40.00% | 0.23 | 0.315 | 136.96% |

**Table 6 - Retention of chelated minerals in yeast**

| Variable | Sample No | Zn (ppm) | | | Mn(ppm) | | |
|---|---|---|---|---|---|---|---|
| | | Exp* | Act** | Rec*** | Exp* | Act** | Rec*** |
| Mineral | DOE2-2 | 1440 | 2164 | 150.3% | 720 | 1017 | 141.3% |
| Combo | DOE2-3 | 1447 | 1404 | 97.0% | 724 | 663 | 91.6% |
| | | | | | | | |

| Variable | Sample No | Fe (ppm) | | | Cu (ppm) | | |
|---|---|---|---|---|---|---|---|
| | | Exp* | Act** | Rec*** | Exp* | Act** | Rec*** |
| Mineral | DOE2-2 | 3600 | 1317 | 36.6% | 360 | 351 | 97.5% |
| Combo | DOE2-3 | 3613 | 2727 | 75.5% | 362 | 252 | 69.6% |

| | | | | | | | |
|---|---|---|---|---|---|---|---|
| * = Expected recovery (if no losses) ** = Actual amount recovered *** = % recovery (retention) | | | | | | | |

As can be seen in Table 5, the retention values for thiamine were consistent with those observed in Example 1 (i.e. there was 100% recovery). (Values over 100% are likely to be attributable to experimental error.) The retention values for vitamin C were lower (approximately 40%) than those for vitamin B1. However, the values are typical of other methods of vitamin C fortification (for example, fortifying with vitamin C post-extrusion). The retention of the vitamins is not affected by the presence of minerals, indicating that there are no compatibility issues.

As can be seen in Table 6, the retention values for the chelated minerals are considerably higher than observed for their unchelated counterparts in Example 2. For example, in the absence of vitamins, the retention of copper increased from 79.1% to 97.5%, the retention of manganese increased from 77.5% to ∼100%, the retention of zinc increased from 40% to 100% and the retention of iron increased from ∼16% to ∼37%. The retention of zinc, manganese and copper was less effective in the presence of vitamins ("Combo" in Table 6), although still at an acceptable level.

## Claims

1. A method of preparing nutritionally fortified yeast comprising:
a) incubating yeast with at least one fermentable substrate under conditions suitable for achieving fermentation;
b) isolating the yeast after fermentation;
c) combining the isolated yeast with one or more chelated minerals and at least one micronutrient selected from vitamins, amino acids and antioxidants to obtain nutritionally fortified yeast; and
d) drying the nutritionally fortified yeast.

2. The method of claim 1, wherein the vitamins comprise, vitamin C, vitamin D, vitamin B1(thiamine), vitamin B2 (riboflavin), vitamin B3 (niacin), vitamin B5 (pantothenic acid), vitamin B6 (pyridoxine), vitamin B9 (folic acid), vitamin B12 (cobalamin), and choline, or
wherein the minerals in the chelated minerals comprise calcium, potassium, sodium, magnesium, iron, copper, manganese, zinc, iodine, and cobalt, or
wherein the amino acids comprise threonine, isoleucine, lysine, methionine, cystine, phenylalanine, tyrosine, valine, arginine, histidine, alanine, and aspartic acid, or
wherein the antioxidants comprise taurine, lipoic acid, glutathione, N-acetyl cysteine, vitamin C and beta-carotene.

3. The method of any preceding claim, wherein the isolated yeast is combined with at least one heat-labile vitamin consisting of vitamin B1 (thiamine) and/or vitamin C; optionally wherein the isolated yeast is combined with at least one chelated mineral selected from iron, zinc, copper, and manganese.

4. The method of any preceding claim wherein the isolated yeast comprises yeast cream, optionally wherein the yeast cream comprises 15 wt.% to 25 wt.% dry matter.

5. The method of any preceding claim, wherein the isolated yeast is combined with at least one vitamin in a total amount of 0.5 wt.% 3 wt.% vitamins by total weight of the solid content of the isolated yeast, optionally in a total amount of 1 wt.% 1.5 wt.% vitamins by total weight of the solid content of the isolated yeast.

6. The method of any preceding claim, wherein the isolated yeast is combined with at least one chelated mineral in a total amount of 0.1 wt.% 2 wt.% minerals by total weight of the solid content of the isolated yeast, optionally in a total amount of 0.5 wt.% to 1 wt.% minerals by total weight of the solid content of the isolated yeast.

7. The method of any preceding claim, wherein the isolated yeast is combined with the one or more chelated minerals and at least one micronutrient at a temperature of 3°C to 45°C for a period of 0.5 hours to 48 hours, optionally wherein the yeast is isolated by centrifugation.

8. The method of any preceding claim, wherein the isolated yeast is allowed to autolyse prior to combining with the one or more chelated minerals and at least one micronutrient, optionally wherein the autolysed yeast comprises 10 wt.% to 25 wt.% dry matter.

9. The method of any preceding claim, wherein the nutritionally fortified yeast is heated to a temperature of 60°C to 135°C.

10. The method of any preceding claim, wherein the nutritionally fortified yeast is dried to a moisture content of 1 wt.% to 10 wt.%, optionally wherein the yeast is dried by spray-drying or freeze-drying.

11. The method of any preceding claim, wherein the yeast is Torula yeast (*Torulopsis utilis, Torula utilis* or *Candida utilis*).

12. The method of any preceding claim, wherein the isolated yeast is combined with at least one vitamin and chelated mineral; or the isolated yeast is combined with at least one chelated mineral, in the absence of any vitamins.

13. The method of any of claim 1, wherein the chelant comprises a peptide or partially hydrolysed peptide, optionally wherein the one or more chelated minerals is selected from a proteinate of calcium, potassium, sodium, magnesium, iron, copper, manganese, zinc, iodine, cobalt and selenium.

14. The method of any preceding claim, wherein the fermentable substrate comprises ethanol.

15. Use of a nutritionally fortified yeast composition obtained by the method of any preceding claim as a palatability enhancer.

## Patentansprüche

1. Verfahren zum Herstellen von mit Nährstoffen angereicherter Hefe, umfassend:
a) Inkubieren von Hefe mit mindestens einem fermentierbaren Substrat unter Bedingungen, die zum Erreichen von Fermentierung geeignet sind;
b) Isolieren der Hefe nach dem Fermentieren;
c) Kombinieren der isolierten Hefe mit einem oder mehreren chelatisierten Mineralien und mindestens einem Mikronährstoff ausgewählt unter Vitaminen, Aminosäuren und Antioxidantien, um eine mit Nährstoffen angereicherte Hefe zu erhalten; und
d) Trocknen der mit Nährstoffen angereicherten Hefe.

2. Verfahren nach Anspruch 1, wobei die Vitamine Vitamin C, Vitamin D, Vitamin B1 (Thiamin), Vitamin B2 (Riboflavin), Vitamin B3 (Niacin), Vitamin B5 (Pantothensäure), Vitamin B6 (Pyridoxin), Vitamin B9 (Folsäure), Vitamin B12 (Cobalamin) und Cholin umfassen, oder
wobei die Mineralien in den chelatisierten Mineralien Calcium, Kalium, Natrium, Magnesium, Eisen, Kupfer, Mangan, Zink, Jod und Kobalt umfassen, oder
wobei die Aminosäuren Threonin, Isoleucin, Lysin, Methionin, Cystin, Phenylalanin, Tyrosin, Valin, Arginin, Histidin, Alanin und Asparaginsäure umfassen, oder
wobei die Antioxidantien Taurin, Liponsäure, Glutathion, N-Acetylcystein, Vitamin C und Beta-Carotin umfassen.

3. Verfahren nach einem vorhergehenden Anspruch, wobei die isolierte Hefe mit mindestens einem wärmelabilen Vitamin kombiniert wird, das aus Vitamin B1 (Thiamin) und/oder Vitamin C besteht; wahlweise wobei die isolierte Hefe mit mindestens einem chelatisierten Mineral ausgewählt unter Eisen, Zink, Kupfer und Mangan kombiniert wird.

4. Verfahren nach einem vorhergehenden Anspruch, wobei die isolierte Hefe Hefecreme umfasst, wobei wahlweise die Hefecreme 15 Gew.-% bis 25 Gew.-%^ Trockensubstanz umfasst.

5. Verfahren nach einem vorhergehenden Anspruch, wobei die isolierte Hefe mit mindestens einem Vitamin in einer Gesamtmenge von 0,5 Gew.-% bis 3 Gew.-% Vitaminen, auf das Gesamtgewicht des Feststoffgehalts der isolierten Hefe bezogen, wahlweise in einer Gesamtmenge von 1 Gew.-% bis 1,5 Gew.-% Vitaminen, auf das Gesamtgewicht des Feststoffgehalts der isolierten Hefe bezogen, kombiniert wird.

6. Verfahren nach einem vorhergehenden Anspruch, wobei die isolierte Hefe mit mindestens einem chelatisierten Mineral in einer Gesamtmenge von 0,1 Gew.-% bis 2 Gew.-% Mineralien, auf das Gesamtgewicht des Feststoffgehalts der isolierten Hefe bezogen, wahlweise in einer Gesamtmenge von 0,5 Gew.-% bis 1 Gew.-% Mineralien, auf das Gesamtgewicht des Feststoffgehalts der isolierten Hefe bezogen, kombiniert wird.

7. Verfahren nach einem vorhergehenden Anspruch, wobei die isolierte Hefe mit dem einen oder den mehreren chelatisierten Mineralien und mindestens einem Mikronährstoff bei einer Temperatur von 3 °C bis 45 °C für eine Zeitspanne von 0,5 Stunden bis 48 Stunden kombiniert wird, wobei die Hefe durch Zentrifugieren isoliert wird.

8. Verfahren nach einem vorhergehenden Anspruch, wobei der isolierten Hefe gestattet wird, sich vor dem Kombinieren mit dem einen oder den mehreren chelatisierten Mineralien und mindestens einem Mikronährstoff zu autolysieren, wobei die autolysierte Hefe wahlweise 10 Gew.-% bis 25 Gew.-% Trockensubstanz umfasst.

9. Verfahren nach einem vorhergehenden Anspruch, wobei die mit Nährstoffen angereicherte Hefe auf eine Temperatur von 60 °C bis 135 °C erhitzt wird.

10. Verfahren nach einem vorhergehenden Anspruch, wobei die mit Nährstoffen angereicherte Hefe bis auf einen Feuchtigkeitsgehalt von 1 Gew.-% bis 10 Gew.-% getrocknet wird, wobei die Hefe wahlweise durch Sprühtrocknen oder Gefriertrocknen getrocknet wird.

11. Verfahren nach einem vorhergehenden Anspruch, wobei die Hefe Torula-Hefe (*Torulopsis utilis, Torula utilis* oder *Candida utilis*) ist.

12. Verfahren nach einem vorhergehenden Anspruch, wobei die isolierte Hefe mit mindestens einem Vitamin und einem chelatisierten Mineral kombiniert wird; oder die isolierte Hefe in Abwesenheit irgendwelcher Vitamine mit mindestens einem chelatisierten Mineral kombiniert wird.

13. Verfahren nach einem von Anspruch 1, wobei das Chelatisierungsmittel ein Peptid oder teilweise hydrolysiertes Peptid umfasst, wobei wahlweise das eine oder die mehreren chelatisierten Mineralien von einem Proteinat von Calcium, Kalium, Natrium, Magnesium, Eisen, Kupfer, Mangan, Zink, Jod, Kobalt und Selen ausgewählt wird.

14. Verfahren nach einem vorhergehenden Anspruch, wobei das fermentierbare Substrat Ethanol umfasst.

15. Verwendung einer mit Nährstoffen angereicherten Hefezusammensetzung, die durch das Verfahren nach einem vorhergehenden Anspruch erhalten wird, als Schmackhaftigkeitsverbesserungsmittel

## Revendications

1. Procédé de préparation de levure nutritionnellement fortifiée comprenant:
a) l'incubation de levure avec au moins un substrat fermentescible sous des conditions convenant à l'atteinte de la fermentation;
b) l'isolement de la levure après la fermentation;
c) la combinaison de la levure isolée avec un ou plusieurs minéraux chélatés et au moins un micronutriment sélectionné parmi les vitamines, les acides aminés et les antioxydants pour obtenir de la levure nutritionnellement fortifiée; et
d) le séchage de la levure nutritionnellement fortifiée.

2. Procédé selon la revendication 1, les vitamines comprenant, la vitamine C, la vitamine D, la vitamine B1 (thiamine), la vitamine B2 (riboflavine), la vitamine B3 (niacine), la vitamine B5 (acide pantothénique), la vitamine B6 (pyridoxine), la vitamine B9 (acide folique), la vitamine B12 (cobalamine), et la choline, ou
les minéraux dans les minéraux chélatés comprenant le calcium, le potassium, le sodium, le magnésium, le fer, le cuivre, le manganèse, le zinc, l'iode, et le cobalt, ou
les acides aminés comprenant la thréonine, l'isoleucine, la lysine, la méthionine, la cystine, la phénylalanine, la tyrosine, la valine, l'arginine, l'histidine, l'alanine, et l'acide aspartique, ou
les antioxydants comprenant la taurine, l'acide lipoïque, la glutathione, la N-acétylcystéine, la vitamine C et le bêta-carotène.

3. Procédé selon l'une quelconque des revendications précédentes, la levure isolée étant combinée à au moins une vitamine thermolabile constituée de la vitamine B1 (thiamine) et/ou de la vitamine C; optionnellement dans lequel la levure isolée est combinée à au moins un minéral chélaté sélectionné parmi le fer, le zinc, le cuivre et le manganèse.

4. Procédé selon l'une quelconque des revendications précédentes dans lequel la levure isolée comprend la crème de levure, optionnellement la crème de levure comprenant de 15 % en pds à 25 % en pds de matière sèche.

5. Procédé selon l'une quelconque des revendications précédentes, dans lequel la levure isolée est combinée à au moins une vitamine en une quantité totale de 0,5 % en pds à 3 % en pds de vitamines par poids total de la teneur solide de la levure isolée, optionnellement en une quantité totale de 1 % en pds à 1,5 % en pds de vitamines par poids total de la teneur solide de la levure isolée.

6. Procédé selon l'une quelconque des revendications précédentes, dans lequel la levure isolée est combinée à au moins un minéral chélaté en une quantité totale de 0,1 % en pds à 2 % en pds de minéraux par poids total de la teneur solide de la levure isolée, optionnellement en une quantité totale de 0,5 % en pds à 1 % en pds de minéraux par poids total de la teneur solide de la levure isolée.

7. Procédé selon l'une quelconque des revendications précédentes, dans lequel la levure isolée est combinée audit un ou plusieurs minéraux chélatés et au moins un micronutriment à une température de 3°C à 45°C durant une période de 0,5 heure à 48 heures, optionnellement la levure étant isolée par centrifugation.

8. Procédé selon l'une quelconque des revendications précédentes, dans lequel la levure isolée est laissée à autolyse avant la combinaison avec lesdits un ou plusieurs minéraux chélatés et au moins un micronutriment, optionnellement dans lequel la levure autolysée comprend de 10 % en pds à 25 % en pds de matière sèche.

9. Procédé selon l'une quelconque des revendications précédentes, dans lequel la levure nutritionnellement fortifiée est chauffée à une température de 60°C à 135°C.

10. Procédé selon l'une quelconque des revendications précédentes, dans lequel la levure nutritionnellement fortifiée est séchée à une teneur en humidité de 1 % en pds à 10 % en pds, dans lequel optionnellement la levure est séchée par séchage par atomisation ou lyophilisation.

11. Procédé selon l'une quelconque des revendications précédentes, dans lequel la levure est la levure Torula (*Torulopsis utilis, Torula utilis* ou *Candida utilis*).

12. Procédé selon l'une quelconque des revendications précédentes, dans lequel la levure isolée est combinée à au moins une vitamine et un minéral chélaté; ou la levure isolée est combinée à au moins un minéral chélaté, en l'absence de quelconques vitamines.

13. Procédé selon l'une quelconque de la revendication 1, dans lequel l'agent chélatant comprend un peptide ou un peptide partiellement hydrolysé, optionnellement dans lequel les un ou plusieurs minéraux chélatés sont sélectionnés parmi un protéinate de calcium, de potassium, de sodium, de magnésium, de fer, de cuivre, de manganèse, de zinc, d'iode, de cobalt et de sélénium.

14. Procédé selon l'une quelconque des revendications précédentes, le substrat fermentescible comprenant de l'éthanol.

15. Utilisation d'une composition de levure nutritionnellement fortifiée, obtenue par le procédé selon l'une quelconque des revendications précédentes, comme agent d'amélioration de la sapidité.
